(19) Europäisches Patentamt
European Patent Office
Office européen des brevets

(11) **EP 0 945 510 A1**

(12) **EUROPEAN PATENT APPLICATION**
published in accordance with Art. 158(3) EPC

(43) Date of publication:
29.09.1999 Bulletin 1999/39

(51) Int. Cl.$^6$: **C12N 15/32**, C12N 1/21,
C07K 14/325, A01N 63/00,
A01H 1/00, C12N 5/00,
A01H 5/00

(21) Application number: 97947868.2

(22) Date of filing: 10.12.1997

(86) International application number:
PCT/JP97/04530

(87) International publication number:
WO 98/26073 (18.06.1998 Gazette 1998/24)

(84) Designated Contracting States:
AT BE CH DE DK ES FI FR GB GR IE IT LI LU MC
NL PT SE

(30) Priority: 10.12.1996 JP 32930996
08.10.1997 JP 27549797

(71) Applicant:
MEIJI SEIKA KAISHA LTD.
Chuo-ku Tokyo 104 (JP)

(72) Inventors:
• OYAMA, Kazuhiko,
Pharmaceutical Research Center
Yokohama-shi, Kanagawa 222 (JP)
• IMAMURA, Keiichi,
Pharmaceutical Research Center
Yokohama-shi, Kanagawa 222 (JP)

• MIDOH, Naoki,
Pharmaceutical Research Center
Yokohama-shi, Kanagawa 222 (JP)
• TANINO, Shigeki,
Pharmaceutical Research Center
Yokohama-shi, Kanagawa 222 (JP)
• KATOH, Akiko,
Pharmaceutical Research Center
Yokohama-shi, Kanagawa 222 (JP)
• IWATA, Michiaki,
Pharmaceutical Research Center
Yokohama-shi, Kanagawa 222 (JP)

(74) Representative:
Lewin, John Harvey
Elkington and Fife,
Prospect House,
8 Pembroke Road
Sevenoaks, Kent TN13 1XR (GB)

(54) **STRAIN BELONGING TO THE GENUS $i(BACILLUS) AND INSECTICIDAL PROTEINS**

(57) An objective of the present invention is to provide Bt bacteria having insecticidal activity not only against insects of the Lepidoptera but also against other insects, an insecticidal protein produced by said Bt bacteria, the genes encoding the protein, hosts to produce the insecticidal protein, and insect pest exterminating agents. The new strain according to the present invention is strain SSK-10, which is classified into Bacillus thuringiensis, deposited under the access number of FERM BP-6162. The protein according to the present invention comprises (a) an amino acid sequence depicted in SEQ ID NO: 1, or (b) an amino acid sequence depicted in SEQ ID NO: 2 which has insecticidal activity wherein one or more amino acids are substituted, deleted, added or inserted.

F I G. I

**Description**

BACKGROUND OF THE INVENTION

Field of the Invention

The present invention relates to a new strain of <u>Bacillus thuringiensis</u>, which is effective in exterminating insect pests, crystalline protein inclusions produced by bacteria of this strain, a insecticidal protein which comprises said inclusions, and a gene encoding this protein. Further, the present invention relates to insect exterminating agents and a method of exterminating insects.

Background Art

Microbial insecticides have a lesser impact on the natural environment than do synthetic chemical insecticides and are considered to be an effective alternative to synthetic chemical insecticides to control insect pests. In fact, since early 1960, microbial insecticides (hereinafter referred to as "Bt agents") containing bacterial cells of <u>Bacillus thuring-iensis</u> (hereinafter referred to as "Bt bacteria"), or crystalline inclusions (hereinafter referred to as "CIs") produced by these bacteria, as an active ingredient, have been used to control various insects belonging to the Leptidoptera.

Bt bacteria are gram-positive bacilli and produce CIs in the cells during their spore-forming phase (H. Hofte and H.R. Whiteley; Microbiol. Rev., 53, 242, 1989). Insects ingesting certain CIs to which they are sensitive will cease their ingestion within several hours then die. Because such action by CIs is specific to insects, Bt agents have no toxicity to other animals and plants, and are thus considered to be a very safe pesticide.

Insecticidal proteins which comprise CIs are produced by the transcription and translation of genes encoded in plasmids or genome DNAs of Bt bacteria. In excess of a hundred genes encoding insecticidal proteins have been isolated to date, but the insecticidal activities of said encoded proteins are known to differ as a function of their amino acid sequences (P. A. Kumar et al., Advances in Applied Microbiology, 42, 1, 1996). Accordingly, a strain of Bt bacteria having a new insecticidal activity is deemed to possess a new insecticidal protein gene.

<u>Bacillus thuringiensis</u> serovar. <u>kurstaki</u> HD-1 (hereinafter referred to as "HD-1") (Microbial Control of Pest and Plant Disease 1970-1980, edited by H.D. Burges, Academic Press, pp. 35-44, 1981) is the Bt strain most commonly used in commercial Bt agents.

Bt agents containing HD-1 as an active component have been effective in exterminating small size insects of the order Lepidoptera, such as <u>Plutella xylostella</u>, which have developed a marked resistance to synthetic agricultural chemicals. However, there are recent reports of the detection of the field strains of <u>Plutella xylostella</u> which have developed a resistance to HD-1. This poses a serious problem in the control of insects.

Furthermore, <u>Spodoptera litura</u> large insects of the Noctuidae, which is classified into the Lepidoptera, are difficult to control because of their low sensitivity to synthetic insecticides, and can cause devastating damages to plants. Nor, in fact, can insects such as <u>Spodoptera litura</u> of the Noctuidae be effectively controlled by conventional Bt agents, and the development of effective Bt agents has been desired.

Conceivable reasons that insects of the Noctuidae, particularly Spodoptera sp. are extremely insensitive to Bt agents are as follows:

(1) Spodoptera sp. have no receptors which can bind to the insecticidal proteins in CIs (Shuichiro Inagaki, Kagaku to Seibutsu, 30, 74, 1992).
(2) proteins in CIs are digested, adsorbed, or inactivated in the digestive juices of Spodoptera making it impossible for said proteins to reach the target receptors.

As such, the development of novel CIs having insecticidal activity against noctuids has been desired.

Most Bt bacteria isolated from the natural environment have no insecticidal activity against insects of the Lepidoptera or any other insects. Very few strains exhibit practical insecticidal activity to control targeted insects, probably because CIs produced by at bacteria kill said insects through the following process:

(1) insects ingest CIs,
(2) the CIs dissolve in the digestive tract to produce a protoxin (insecticidal protein),
(3) the protoxin is activated by an enzymes to a toxin (processing step),
(4) the activated toxin (processed insecticidal protein) is bound to receptors on mid-gut cells of the insects, and micropores are formed, and
(5) the mid-gut cells of the insects decompose.

The environment of the digestive tract varies among insects and is closely related to the diet of the insects. Hence the inactivation or activation of CIs, which become insecticidal only when insects ingest them with other food, also differs among insects. It is believed, therefore, that only those CIs for which the steps (1) to (5) can be completed in the mid-gut of an insect will have insecticidal activity against that insect.

On the other hand, spores, one of the phases in the life cycle of Bt bacteria, are known to have a marked synergistic effect on the insecticidal activity of CIs. R. Milne et al (Analytical Chemistry of Bacillus thuringiensis, edited by L. A. Hickle, American Chemical Society, p22, 1990) have reported that the insecticidal activities of two kinds of CIs are reversed due to the presence of spores. W. J. Moar et al. (Appl. Environ. Microbiol., 61, 2086, 1995) have reported that insects which have ingested only CIs produced by Bt bacteria readily become much more resistant than those which have ingested a mixture of CIs and spores. These reports indicate that two components produced by Bt bacteria, namely CIs and spores, are closely intertwined in their interaction with insects. Hence researchers are focusing on the role of spores in suppressing the development of a resistance to Bt agents among insects.

SUMMARY OF THE INVENTION

The present inventors have now successfully isolated a strain of Bt bacteria which is effective in controlling a broad spectrum of insects. This strain is highly effective in controlling insects, including those of the family Noctuidae.

Also, the present inventors have found that the Bt strain produces CIs which are more specifically soluble and stable under alkaline conditions (pH 9 to 10) as compared to those of strain HD-1; that CIs alone exhibit a high level of insecticidal activity against insects of Spodoptera litura and Plutella xylostella; that a combination of CIs and spores exhibits a much better insecticidal activity against Spodoptera litura than CIs alone; and that insecticidal activity can be regenerated with the coexistence of solubilized CIs and spores.

Furthermore, the present inventors have identified the amino acid sequence of the insecticidal protein produced by Bt bacteria of this strain, and have isolated the gene encoding this amino acid sequence.

Accordingly, an objective of the present invention is to provide Bt bacteria which has insecticidal activity not only against insects of the order Lepidoptera but also against other insects. Another objective of the present invention is to provide an insecticidal protein produced by said Bt bacteria, a gene encoding said protein, a vector possessing said gene, and a host possessing said gene.

Another objective of the present invention is to provide transformed plants which are capable to produce an insecticidal protein, an agent for exterminating insect pests, and a method for exterminating insect pests.

The novel strain according to the present invention is strain SSK-10, which was deposited with the access number of FERM BP-6162 at the National Institute of Bioscience and Human-Technology Agency of Industrial Science and Technology. This strain is classified into Bacillus thuringiensis.

The insecticidal protein according to the present invention has the following amino acid sequence (a) an amino acid sequence of SEQ ID NO: 2, or (b) an amino acid sequence of SEQ ID NO: 2 which has an insecticidal activity wherein one or more amino acids in SEQ ID NO: 2 are substituted or deleted, or one or more amino acids are added or inserted into the amino acid sequence of SEQ ID NO: 2.

Deposition of the Microorganism

The new strain SSK-10 according to the present invention was deposited at the National Institute of Bioscience and Human-Technology Agency of Industrial Science and Technology (1-3 Higashi 1-Chome, Tsukuba city, Ibaraki, Japan) dated November 12, 1996. The access number is FERM BP-6162.

BRIEF DESCRIPTION OF THE DRAWING

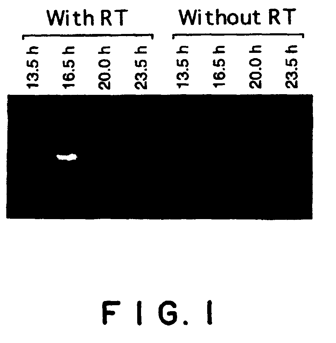

Figure 1 illustrates the results of an assay on transcription of the gene of the insecticidal protein according to the present invention. With RT: a reaction solution with RT was used. Without RT: a reaction solution without RT was used.

DETAILED DESCRIPTION OF THE INVENTION

Microorganisms

The strain SSK-10 was isolated from household dust in Sakado city, Saitama Prefecture. Its characteristics are shown in Table 1.

Table 1

| Colony features: Large colonies typical to Bt bacteria, with a colorless surface. | | |
|---|---|---|
| Cell morphology in the growth phase: Typical to Bt bacteria. | | |
| Morphological features: Mobile bacilli (1.0 to 1.2 $\mu$ x 3.0 to 5.0 $\mu$). | | |
| Gram staining: Positive. | | |
| Spore morphology: Oval. A marked swelling of sporangium caused by spores is not observed. | | |
| Serotype of flagella: Classified as H7 (or serovar. aizawai)*. | | |
| Intracellular content: Large bi-pyramidal crystalline protein inclusions (which have a molecular weight of about 130 kDa as determined by SDS-PAGE). | | |
| Insecticidal protein: Classified as Cry9n. A homology search showed only a 71% homology to even the most homologous Cry9Cal (Appl. Environ. Microbiol., 62, p80, 1996). | | |
| Aerobic/anaerobic features: Facultative anaerobic bacteria. | | |
| Biochemical features: | Catalase reaction | Positive |
| | Lecithinase reaction | Positive |
| | VP reaction | Positive |

* Preparation of H serum and classification of the strain were carried out according to M. Ohba and K. Aizawa, J. Invertebr. Pathol., 15, 139-140, 1978.

Features which are very characteristic of the CIs and spores produced by the new strain of the present invention are explained below in further detail.

J.D. Tang (Appl. Environ. Microbiol., 62, 564, 1996) has reported that of the CryI insecticidal protein, CryIC is known to be particularly effective against noctuids of the family Noctuidae but its insecticidal activity against insects of Plutella xylostella is only about 1/10 of that of CryIA(a) produced by strain HD-1. Also, the insecticidal activity of Bt bacteria containing CryIC and CryIA, as disclosed in Japanese Patent Laid-open Publication No. 509235/1993, is three times higher against Spodoptera frugiperda and two to several times higher against Plutella xylostella than that of the bacteria of strain HD-1. In contrast, the insecticidal activity of CIs produced by cells of strain SSK-10 is at least 30 times greater against Spodoptera litura and at least five times greater against Plutella xylostella than that of the CIs produced by cells of strain HD-1. Furthermore, the CIs of strain SSK-10 have more specific solubility and higher stability than the CIs of strain HD-1 under alkaline conditions (pH 9 to 10).

Further, a homology search (GenBank, EMBL, DDBJ, PDB sequences) of the base sequence of the insecticidal protein gene from strain SSK-10 showed little homology to genes of known Bt insecticidal protein. Only a 71% homology was found at the amino acid level with even the most homologous Cry9Cal (B. Lambert et al., Appl. Environ. Microbiol., 62, 80, 1996). The Bt insecticidal protein is produced as a protoxin with a molecular weight of 130 kd to 140 kd, which is then digested into a toxin of 60 kd to 70 kd in the mid-gut of insects. It is known that, in fact, this toxin has the insecticidal activity and that the C-terminal site of the protoxin is not required (H. Hofte and H.R. Whiteley, Microbiol. Rev. 53, 242, 1989). Thus, a homology search (GenBank CDS translations, PDB, SwissProt, PIR) was performed on a limited amino acid sequence, namely from the 1st to the 700th amino acid at the N-terminal site required for insecticidal activity. Only a 60% homology of the insecticidal protein gene to even the most homologous Cry9Cal was observed, clearly indicating that this gene is a novel gene.

These findings indicated that the strain SSK-10 is a new bacterial strain which produces a novel CI.

Furthermore, the insecticidal activity is enhanced by combining the spores produced by cells of strain SSK-10 with the CIs, and the insecticidal activity is restored by combining solubilized CIs (hereinafter referred to as SCIs) which by themselves have no insecticidal activity with the spores. Further, it was found that these actions could be attained to a substantial degree with the same spore/CI ratio as found in an ordinary culture. Thus the use of CIs (or SCIs) and spores produced by the new strain SSK-10 of the present invention can provide a highly effective Bt agent for the control of insect pests.

Cells of strain SSK-10 of the present invention can be cultured under ordinary conditions. For example, incubation can be carried out using a medium containing a carbon source such as glucose and a nitrogen source such as soybean powder at an appropriate culture temperature between 20 and 40 °C. Incubation is preferably carried out under aerobic conditions while stirring with aeration for 1 to 4 days.

EP 0 945 510 A1

## Insecticidal protein and its gene

The present invention provides an insecticidal protein produced by the cells of strain SSK-10.

The insecticidal protein according to the present invention has an amino acid sequence of SEQ ID NO: 2. Further, the insecticidal protein according to the present invention may have an amino acid sequence of SEQ ID NO: 2 which has insecticidal activity wherein one or more (e.g., one to several) amino acids are added or inserted into the amino acid sequence of SEQ ID NO: 2, or one or more (e.g., one to several) amino acids in SEQ ID NO: 2 are replaced or deleted. It will be appreciated that those skilled in the art can add, insert, replace or delete an amino acid(s) in the amino acid sequence of SEQ ID NO: 2 without any special difficulty by referring to the sequence of SEQ ID NO: 2.

In the present invention, the expression "protein which has insecticidal activity" denotes a protein which is evaluated by those skilled in the art to be insecticidal; for example, a protein which is evaluated to be insecticidal in an experiment carried out under the same conditions as in Test Examples 1 to 3 and 5 to 7.

The insecticidal protein according to the present invention has insecticidal activity against the Lepidoptera, Coleoptera and/or Diptera as shown in the Test Examples. In particular, it is highly insecticidal against Spodoptera litura and Plutella xylostella classified into the Noctuidae, the Lepidoptera.

Examples of insects which are classified into the Lepidoptera include Spodoptera litura, Spodoptera exigua, Mamesta brassicae, Plutella xylostella, Hyphantria cunea, Adoxophyes sp., Autographa nigrisigna, and Pieris rapae.

Examples of insects which are classified into the Coleoptera include Anomala cuprea, Popillia japonica, Lissorhoptrus oryzophilus, and Aulacophora femoralis.

Examples of insects which are classified into the Diptera include Aedea aegypti and Culex piniens.

The insecticidal protein according to the present invention may be in the form of crystalline protein inclusions. The protein in such form may be obtained by expressing the insecticidal protein of the present invention in microorganisms, in particular in transformed Escherichia coli, or by culturing cells of the new strain SSK-10 of the present invention. A method for expression in Escherichia coli will be described below.

The insecticidal protein according to the present invention may be in the form of solubilized crystalline protein inclusions. The protein in such form can be obtained by dissolving the protein of the present invention in an alkaline solvent.

The present invention provides nucleotide sequences encoding the insecticidal protein of the present invention. A typical sequence of this nucleotide sequence has the DNA sequence, in whole or in part, of SEQ ID NO: 1.

When the amino acid sequence of the insecticidal protein of the present invention is given, the nucleotide sequences encoding the protein are readily determined, and various nucleotide sequences encoding the amino acid sequence of SEQ ID NO: 2 can be selected. Thus, a nucleotide sequence encoding the insecticidal protein according to the present invention include DNA sequences which degenarate as a result of the genetic code as to the DNA sequence of SEQ ID No.1 as well as RNA sequences corresponding to the DNA sequences.

The nucleotide sequences of the present invention may be a natural derivative, wholly synthetic, or semi-synthetic using partial natural sequences.

The gene encoding the insecticidal protein of the present invention can be isolated from strain SSK-10, for example, by the following method. First, DNA is extracted from the cells of strain SSK-10 and cleaved with appropriate restriction enzymes, after which a library comprising DNA of strain SSK-10 is constructed using a phage vector or a plasmid vector. Appropriate primers are synthesized from the nucleotide sequence of SEQ ID NO: 1, then PCR, using the SSK-10 derived DNA as a template, is performed to amplify a DNA fragment of the gene. Said DNA fragment is then used as a probe to screen the library in order to isolate the whole DNA length of the gene.

Alternatively, the gene may be isolated by preparing an antibody for the insecticidal protein of the present invention, and then screening a genomic library or a cDNA library using this antibody.

Further, the total length of DNA of the novel gene may be isolated by synthesizing 5' terminal and 3' terminal primers of the open reading frame of the gene encoding the insecticidal protein of the present invention using DNA derived from strain SSK-10 as a template, and using them to carry out PCR amplification.

Also, the total length of DNA of the gene may be obtained by screening the chromosomal library or the cDNA library using conventional methods used in the field of genetic engineering; for example, using an appropriate DNA probe constructed based on information from a partial amino acid sequence.

Mutations may be introduced into the gene, for example, by using a commercial mutagenesis kit, or by PCR using primers containing mutations ("PCR and Its Application," edited by Yoshiyuki Sakakibara et al., Yodosha, pp63-67, 1990).

The present invention provides a vector in which the nucleotide sequence of the present invention exists in such a mode that it is replicable in a host cell and the protein encoded by the nucleotide sequence may be expressed.

Furthermore, the present invention provides a host cell transformed by the vector. This host-vector system is not particularly limited and, for example, an expression system of a protein fused with another protein may be used.

Examples of the vectors include plasmid vectors such as Ti plasmid vector.

5

In order to express the target protein by introducing the vector into a host cell, the vector of the present invention may have sequences to control the expression (e.g., a promoter sequence, a terminator sequence, an enhancer sequence, a transcription regulating region, and a SD sequence), gene markers to select host cells (e.g., a neomycin resistant gene and a kanamycin resistant gene), in addition to the nucleotide sequence of the present invention. Further, the vector may have the nucleotide sequence of the present invention in a repeated form (for example, in a tandem form). The sequences may be placed in the vector by any ordinary method, and the transformation of host cells by this vector may be carried out using a conventional method used in this field. The vector of the present invention may be constructed using methods and techniques customarily used in the field of genetic engineering.

Examples of the host cells include microorganisms (for example, gram-negative bacteria such as Escherichia coli and Pseudomonas sp., gram-positive bacteria such as Bacillus sp., and Eumycetes such as fungi and yeasts).

Transformed host cells (for example, E. coli) are cultured in an appropriate medium, and the insecticidal protein of the present invention may be obtained from this culture product. Accordingly, another embodiment of the present invention provides a method for preparing the insecticidal protein of the present invention. The transformed cells may be cultured virtually under the same conditions as those used for the original cells. Further, recovery and purification of the insecticidal protein of the present invention from the culture fluid are carried out by conventional methods.

Thus, a stable and mass supply of the insecticidal protein of this invention may be carried out not only in Bt bacteria but in other heteroorganisms (i.e., microorganisms, plants, etc.) as well. Further, insertion of the nucleotide sequence of the present invention into Bt bacteria other than strain SSK-10 will be considered to enhance insecticidal activity, making it possible to construct transformed Bt strains which can effectively control a broader range of insects.

Furthermore, it will be appreciated by those skilled in the art that one or more foreign Bt insecticidal protein genes may be introduced into strain SSK-10 to enhance insecticidal activity so as to produce strains of transformed Bt bacteria which can effectively control or exterminate a wider range of insects.

According to the present invention, plant cells or plants can be selected as hosts. Therefore, the present invention provides transformed plants which are resistant to insects. Examples of transformed plants include monocotyledonous plants (e.g., rice, corn, wheat, and asparagus) and dicotyledonous plants (e.g., potato, sugar beet, carrot, cauliflower, lettuce, tomato, egg plant, tobacco, melon, cucumber, soy bean, apple, orange, strawberry, poplar, cotton, carnation, petunia, alfalfa, and mint).

Genes may be introduced into plant cells or plants, for example, by a direct gene transfer such as a particle gun, PEG, electroporation and microinjection, or by an indirect gene transfer using a Ti plasmid vector of Agrobacterium. Dicotyledonous plants and their cells can be transformed, for example, by the method of Vaeck M. et al. (Nature, 328, 33-37, 1987), and monocotyledonous plants and their cells can be transformed, for example, by the method of Hong Y. et al. (Scientia Agricultura Sinica, 22, 1-5, 1989). It will be appreciated by those skilled in the art that transformed plant cells can be redifferentiated to obtain complete plants.

The present invention provides seeds of the insect resistant transformed plants. The seeds of the present invention may be obtained from plants obtained by redifferentiation of the transformed plant cells, or from the transformed plants.

### Insect pest exterminating agents and insect pest exterminating methods

The protein according to the present invention has insecticidal activity. Therefore, the present invention may provide insect exterminating agents comprising the insecticidal protein of the present invention as an active ingredient. The term "agent for exterminating insect pests (insect pest exterminating agent)" as used herein includes plant protecting agents, insect controlling agents, insecticides, and insect pest repellents.

In order to enhance the insecticidal activity of the protein in the insect pest exterminating agents of the present invention, it is preferable to use said protein in combination with spores produced by strain SSK-10.

Insecticidal activity of the solubilized crystalline protein inclusions will be regenerated if used in combination with the spores (see Test Examples described later). Hence if the solubilized crystalline protein inclusions are used as the active ingredient, it is preferable that said solubilized crystalline protein inclusions be used in combination with spores. These solubilized crystalline protein inclusions are preferably used for the extermination of insect pests which have less amount of digestive enzymes.

The new strain of the present invention produces the insecticidal protein as described above. Therefore, another embodiment of the present invention provides insect pest exterminating agents comprising the new strain of the present invention.

Another embodiment of the present invention provides insect pest exterminating agents comprising microorganisms so transformed as to produce the insecticidal protein of the present invention.

The insect pest exterminating agents of the present invention may contain either cells of the new strain, the transformed microorganisms, or the insecticidal protein of the present invention, either alone or in any combination thereof.

The insect pest exterminating agents of the present invention may be applied to plants infested or likely infested with insect pests, and may be sprayed in the undiluted or diluted form using water or other suitable diluents.

The insect pest exterminating agents of the present invention may be used to protect a wide range of plants on which insect pests are likely to multiply. Examples of important plants to be protected by the method of the present invention include vegetables such as cabbage, broccoli and other flowering vegetables, corn, cotton, potato, rice, citrus fruits, and deciduous fruit trees. Non-agricultural trees, such as those in streets, parks, afforested lands and natural forests, are also included.

Insect pests which may be combated by the method of the present invention are those mentioned above.

The insect pest exterminating agents of the present invention may be obtained, for example, by concentrating (e.g., by centrifugation or filtration) a culture fluid of strain SSK-10 or a culture fluid of transformed microorganisms in which the gene encoding the insecticidal protein of the present invention is introduced, and then combining said concentrate with desired agents as appropriate (e.g., a surfactant, a wetting agent, a solid diluent, a dispersing agent, and a UV stabilizer) to yield the desired formulation such as a wettable powder, powder, flowable agent, or the like.

If necessary and/or if desired, the insect pest exterminating compositions of the present invention may be combined with various insecticidal agents, bactericidal or fungicidal agents, weed controlling agents, plant growth controlling agents, synergistic agents (including activity enhancing substances contained in the culture supernatants), inducing agents, plant nutrients, fertilizers, or the like, when preparing the formulation or when spraying. Furthermore, the compositions may contain Bt bacteria strains other than that of the present invention, and insecticidal proteins other than the protein of the present invention.

The present invention provides methods for exterminating insect pests by treating plants which have been or could be infested with insect pests with the insecticidal protein of the present invention, cells of the new strain of the present invention, and/or microorganisms so transformed as to produce the insecticidal protein of the present invention.

The term "method for exterminating insect pests" as used herein includes methods of protecting plants from insect pests, methods of controlling insect pests, insecticidal methods, methods of repelling insect pests, or the like.

In exterminating insect pests according to the present invention, the insecticidal protein of the present invention, cells of the new strain of the present invention, and/or microorganisms so transformed as to produce the insecticidal protein of the present invention may be sprayed without further formulations, or the formulations may be sprayed with or without dilution. Insect pests which may be combated and plants which may be protected are the same as described above for insect pest exterminating agents.

EXAMPLES

The present invention is further illustrated by the following Examples which are not intended as a limitation of the invention.

Example 1: Selection of strain SSK-10 of Bt bacteria

Soil and dust samples were collected from all over Japan, and Bt bacteria were isolated from them according to the method described by Ohba et al. (J. Invertebrate Pathology, 47, 12, 1986). Isolated Bt bacteria were cultured on a conventional agar nutrient medium (1.0% meat extract, 1.0% peptone, 0.5% NaCl, 2.0% agar, pH 7.2), then the resulting mixture of spores and CIs was scratched off and suspended in sterile water to prepare a mixed suspension of the spores and CIs of the specified concentration. Sections of cabbage leaves, 5 cm in diameter, were treated with this suspension and fed to various insects to evaluate the insecticidal activity of the Bt bacteria.

As a result, the strain SSK-10, which had the highest insecticidal activity against <u>Spodoptera litura</u>, was selected.

Example 2: Purification of CIs

Cells of strain SSK-10 of the present invention were suspended in sterile water, spread evenly on a conventional agar nutrient medium, and cultured at 28 °C for 5 days. After spore formation and CI production were confirmed, sterile distilled water was added to the plate medium, and the pellets of mixed spores and CIs were recovered by centrifugation and then washed 3 times with sterile distilled water. Next, the resulting pellets were suspended in a small amount of sterile distilled water, and CIs were isolated by the urografin density gradient (40 to 70%) according to the method of R. Milne (J. Invertebrate Pathology, 29, 230, 1977) and washed 3 times with water. The isolated CIs were purified again by the urografin density gradient (60 to 70%) method and then the purified CIs were washed 5 times with water. The isolated CIs were confirmed by a phase-contrast microscope to be at least 99%, then freeze-dried. As a result, 35 mg (dry weight) of CIs were obtained from 1 g (wet weight) of the spore-CI mixture pellet. CIs were prepared in the same manner using strain HD-1 for use as a control.

Example 3: Liquid culture of cells of strain SSK-10

One platinum-loopful of the culture of strain SSK-10 grown on an agar medium was inoculated in a conventional liquid nutrient medium (0.3% meat extract, 0.5% glucose, 0.4% peptone, 0.1% NaCl, pH 7.2, 5 ml/test tube). The test tubes were set in a reciprocating shaker and incubated at 28 °C for 10 to 24 hours. A 0.5 ml portion of the culture fluid thus obtained was inoculated into 100 ml of the conventional liquid nutrient medium in a 500-ml Erlenmeyer flask. The flask was set in a rotary shaker (250 rpm) and incubated at 28 °C for 2 days, after which the culture fluid was centrifuged at 15,000 rpm for 20 minutes to obtain a solid residue of pellets. The pellets were washed 3 times with an appropriate amount of water and centrifuged again at 15,000 rpm for 20 minutes, and then freeze-dried to obtain about 400 mg per flask of a freeze-dried product. The dried products thus obtained were weighed, diluted with distilled water, and then subjected to the tests for insecticidal activity against various insects. A dried product was prepared in the same manner using strain HD-1 for use as a control.

Test Example 1: Insecticidal activity of CI against Spodoptera litura

The CIs prepared by the method of Example 2 were diluted to the specified concentrations, and a wetting agent was added. Cabbage leaf sections were sprayed with the test fluids and dried in plastic cups in air, and then third-instar larvae of Spodoptera litura were freed (5 larvae per cup). The cups were then covered and placed in an incubator at 25 °C. Life or death was observed after 3 days, and median lethal concentrations ($LC_{50}$) were calculated by the Probit method.

Results are shown in Table 2.

Table 2

| Insecticidal activity of CIs against Spodoptera litura | |
|---|---|
| Sample | $LC_{50}$ (ppm) |
| SSK-10 | 7.5 |
| HD-1 (control) | >250 |

This test showed that the insecticidal activity of CIs of strain SSK-10 against Spodoptera litura was more than 30 times higher than that of strain HD-1.

Test Example 2: Insecticidal activity of CIs against Plutella xylostella

The CIs prepared by the method of Example 2 were diluted to the specified concentrations, and a wetting agent was added. Cabbage leaf sections were sprayed with the test fluids and dried in plastic cups in air, and then second-instar larvae of Plutella xylostella were freed (5 larvae per cup). The cups were then covered and placed in an incubator at 25 °C. Life or death was observed after 3 days, and median lethal concentrations ($LC_{50}$) were calculated by the Probit method.

Results are shown in Table 3

Table 3

| Insecticidal activity of CIs against Plutella xylostella | |
|---|---|
| Sample | $LC_{50}$ (ppm) |
| SSK-10 | 0.79 |
| HD-1 (control) | 4.0 |

This test showed that the insecticidal activity of CIs of strain SSK-10 against Plutella xylostella was more than 5 times higher than that of strain HD-1.

Test Example 3: Effect of strain SSK-10 on Bt-agent resistant Plutella xylostella

Insects of Plutella xylostella (Iwaoka line), which are resistant to an existing Bt agent consisting of cells of strain HD-1 as a major component, were caught in Iwaoka District in Hyogo Prefecture. The efficacy of strain SSK-10 against these insects of a resistant line was compared with that against insects of a sensitive line. The dried product prepared by the method of Example 3 was diluted with a solution supplemented with a wetting agent to the specified concentrations. Cabbage leaf sections were sprayed with the test fluids and dried in plastic cups in air, and then second-instar larvae of the Iwaoka line of Plutella xylostella or the sensitive line of Plutella xylostella were freed (5 larvae per cup). The cups were then covered and placed in an incubator at 25 °C, and life or death was observed after 3 days. Toaro-CT wettable powder (Towagosei Chem. KK) was used as the control chemical. Median lethal concentrations ($LC_{50}$) were calculated by the Probit method, and resistance ratios were calculated by dividing the $LC_{50}$ value for Iwaoka line insects by the $LC_{50}$ value for sensitive line insects.

$$\text{Resistance ratio} = \frac{LC_{50} \text{ for Bt-agent resistant insect lines}}{LC_{50} \text{ for Bt-agent sensitive insect lines}}$$

Results are shown in Table 4

Table 4

| Insecticidal activity against Bt-agent resistant Plutella Xylostella | |
| --- | --- |
| Sample | Resistance ratio |
| SSK-10 | 3.59 |
| Toaro-CT wettable powder* (control) | 50.1 |

*Towagosei Chem. KK

Test Example 4: Solubility and Stability of CIs produced by strain SSK-10

The CIs of strains SSK-10 and HD-1 prepared by the method of Example 2 were suspended in a 50 mM $Na_2CO_3$-$NaNCO_3$ solution (pH 9.5) and maintained at 37 °C. After 30 minutes, samples were taken and centrifuged. The suspension before centrifugation and the supernatant after centrifugation were analyzed by SDS polyacrylamide gel electrophoresis. Solubility was calculated by dividing the amount of 130 kd protein in the supernatant by the amount of 130 kd protein in the suspension before centrifugation. Results are shown in Table 5

Table 5

| Solubility of CIs | |
| --- | --- |
| Sample | Mortality (%) |
| SSK-10 | >90 |
| HD-1 | <50 |

Further, samples were taken at specified times from the suspension kept at 37 °C, and the amounts of 130 kd protein were analyzed by SDS-polyacrylamide gel electrophoresis. The amount of the protein immediately after suspension (0 hour) was set as 100, and stability over time was expressed as the percentage of remaining protein. Results are shown in Table 6.

Table 6

| Stability of CIs | | | | |
|---|---|---|---|---|
| Sample | Remaining protein (%) | | | |
| | 0 hr | 2 hrs | 6 hrs | 24 hrs |
| SSK-10 | 100 | 100 | 100 | 15 |
| HD-1 | 100 | 85 | 40 | 0 |

Test Example 5: Insecticidal activity of CIs and SCIs against Spodoptera litura

(1) Preparation of CI solution

A solution supplemented with a wetting agent was added to the CIs of strain SSK-10 prepared by the method of Example 2, and the admixture was diluted to the specified concentration.

(2) Preparation of SCI solution

The CIs of strain SSK-10 prepared by the method of Example 2 were suspended in a 50 mM $Na_2CO_3$-$NaHCO_3$ solution (pH 9.5) and the suspension was kept at 37 °C for 1 hour. After solubilization, the supernatant was obtained by centrifugation, a solution supplemented with a wetting agent was added to the supernatant, and the admixture was diluted to the specified concentration.

(3) Measurement of insecticidal activity and results

Cabbage leaf sections were sprayed with the CI solution and the SCI solution prepared in (1) and (2) above and dried in plastic cups in air, and then third-instar larvae of Spodoptera litura were freed (5 larvae per cup). The cups were then covered and placed in an incubator at 25 °C, and life or death was observed after 3 days.
Results are shown in Table 7.

Table 7

| Insecticidal activity of CIs and SCIs against Spodoptera litura | |
|---|---|
| Sample | Mortality (%) |
| CI (100 ppm) | 100 |
| SCI (100 ppm) | 0 |

Test Example 6: Effect of spores on insecticidal activity against Spodoptera litura

The dried product of strain SSK-10 cells containing the CIs and spores prepared by the method of Example 3 were suspended in a 50 mM $Na_2CO_3$-$NaHCO_3$ solution (pH 9.5) and the suspension was kept at 37 °C for 1 hour. After confirming under a microscope that the CIs were completely dissolved, the supernatant (the fraction containing SCIs) and the precipitate (the fraction containing spores) were obtained by centrifugation. Separately, the CIs obtained by the method of Example 2 were formulated to the same concentration as that of the dried product of strain SSK-10 cells and subjected to the test. Each sample was diluted with a solution supplemented with a wetting agent to the specified concentration, and cabbage leaf sections were sprayed with the sample solutions. The leaf sections were dried in plastic cups in air, and then third-instar larvae of Spodoptera litura were freed (5 larvae per cup). The cups were then covered and placed in an incubator at 25 °C, and life or death was observed after 3 days.
Results are shown in Table 8.

Table 8

| Synergistic effect of spores on insecticidal activity against Spodoptera litura | | | |
|---|---|---|---|
| Sample | CI or SCI(ppm) concentration | Spore(ppm) concentration | Mortality (%) |
| SCI | 25 | 0 | 0 |
| CI | 25 | 0 | 60 |
| SCI+spore | 25 | 75 | 75 |
| CI+spore | 25 | 75 | 100 |
| Spore | 0 | 75 | 0 |

Test Example 7: Insecticidal activity against Culex piniens

The dried product of strain SSK-10 cells prepared by the method of Example 3 was diluted with deionized water to the specified concentration and placed in a petri dish. 20 Culex pipiens larvae 4 days after hatching were freed in the dish and the dish was placed in an incubator at 25 °C.

Life or death was observed after 24 hours and the median lethal concentration ($LC_{50}$) was calculated to be 37.2 ppm by the Probit method. Thus, this test showed that the dried product of strain SSK-10 cells also had insecticidal activity against insects classified into the Diptera.

Example 4: Cloning of gene of novel insecticidal protein produced by strain SSK-10

(1) Construction of genomic library of strain SSK-10

Cells of strain SSK-10 (FERM BP-6162) were cultured overnight on a Luria-Bertani (LB) medium at 28 °C. The cells were collected by centrifugation, and DNA was isolated using ISOPLANT (Nippon Gene) in accordance with the attached manual. The isolated DNA was partially digested with Sau3 A, treated with alkaline phosphatase, then the 5' terminal of the resultant DNA fragment was dephosphorylated. This DNA fragment was inserted into a phage vector, Lambda DASH II (Stratagene). The recombinant phage vector thus obtained was subjected to in vitro packaging using Gigapack III Gold Packaging Extract (Stratagene) in accordance with the attached manual. Next, cells of E. coli XL1-Blue MRA (P2) strain were infected with said recombinant phage, and then cultured on a plate to obtain plaques.

(2) Construction of probes

The insecticidal protein gene of strain SSK-10 was also assumed to have regions which were well conserved among known Bt insecticidal protein genes. Accordingly, base sequences of known Bt insecticidal protein genes were analyzed, and primers corresponding to 2 well-conserved regions (SEQ ID NOS: 3 and 4) were synthesized. A DNA fragment of about 1 kb was amplified by PCR using these primers and the DNA isolated from SSK-10 as the template. This proved that the insecticidal protein gene of strain SSK-10 can be amplified using these primers. In the screening described in (3) below, a fluorescein-labeled DNA fragment which was amplified by the PCR for which fluorescein-11-dUTP (Amersham) was admixed in the reaction solution was used as the probe.

(3) Screening of the novel insecticidal protein gene

A Hybond-N+ membrane (Amersham) was placed on the plate on which the plaques were formed in (1) above, and the plaques adhered to the membrane. This membrane was treated with alkali, the recombinant phage DNA on the membrane was denatured into a single strand, and the denatured DNA was adsorbed onto the membrane. The membrane with the adsorbed phage DNA was placed into a polyethylene bag, a buffer solution prepared using Hybridization Buffer tablets (Amersham) was added, then the bag was sealed and incubated at 65 °C for 1 hour. After adding the fluorescein-labeled probes, which had been denatured by heating, hybridization was carried out overnight at 65 °C. Then, the membrane was washed, and the fluorescein-labeled plaques were labeled with an alkaline-phosphatase-labeled anti-fluorescein antibody. The plaques thus labeled with alkaline phosphatase were treated with nitroblue tetrazolium chloride and X-phosphate for color development, wherein these plaques which were firmly hybridized with the probe were visualized, from which a positive clone was selected. A DNA fragment containing the 5' upper and 3' lower regions homologous to the probe was cleaved from this positive clone using restriction enzymes and subcloned into pBluescript II (Stratagene).

(4) Determination of base sequence

From the recombinant pBluescript II constructed in (3) above, deletion clones, each of which was missing a sequence of about 300bp from the incorporated DNA fragment, were prepared using Deletion Kit for Kilo-Sequence (Takara) in accordance with the attached manual. The sequences of these deletion clones were determined by reacting them with Dye Terminator Cycle Sequencing Ready Reaction (Perkin Elmer) in accordance with the attached manual, followed by an analysis with 373A DNA Sequencer (Perkin Elmer). The base sequence of the DNA fragment of 3867 bp containing the novel insecticidal protein gene of strain SSK-10 thus determined is shown in SEQ ID NO: 1. This base sequence had an ORF of 3453 bp, which encodes a protein of 129.9 kd, pI 4.79, having 1150 amino acids. The amino acid sequence of this protein is shown in SEQ ID NO: 2.

(5) Homology search

Results of a homology search using data bases (GenBank, EMBL, DDBJ. PDB sequences) showed that the base sequence of the novel insecticidal protein gene obtained from strain SSK-10 showed little homology to the genes of known Bt insecticidal proteins, and only a 71% homology to even the most homologous Cry9Cal (B. Lambert et al., Appl. Environ. Microbiol., 62, 80, 1996) at the amino acid level. The Bt insecticidal proteins are produced as a protoxin of 130 kd to 140 kd, which is then digested into toxins of 60 kd to 70 kd in the mid-gut of insects. It is known that, in fact, this toxin has the insecticidal activity and that the C terminal site of the protoxin is not required for the activity (H. Hofte and H.R. Whiteley, Microbiol. Rev., 53, 242, 1989). Therefore, a homology search (GenBank CDS translations, PDB, Swissprot, PIR) was performed on a limited amino acid sequence, namely from the 1st to the 700th amino acid at the N terminal site required for insecticidal. Only a 60% homology to even the most homologous Cry9cal was observed, clearly indicating that this gene is a novel gene.

Example 5: Transcription of novel insecticidal protein gene

(1) Isolation of RNA from strain SSK-10

One loopful of cells of strain SSK-10 were inoculated into 20 ml of LB medium, and incubation was carried out at 28 °C. 0.5 ml of the culture was sampled at 13.0, 16.5, 20.0, 23.5 hours after the start of incubation. CIs were observed under a phase-contrast microscope starting at 16.5 hours after the start of incubation. The sampled culture fluids were immediately centrifuged to collect cells, and RNA was isolated using RNeasy Mini Kit (Qiagen) in accordance with the attached manual. The RNA thus isolated was treated with DNase to decompose any DNA contaminant and then used for analysis by Reverse Transcriptase (RT)-PCR as described below.

(2) Analysis of transcription by RT-PCR

Sequences of SEQ ID NO: 5 (corresponding to the amino acids 461 to 480 of SEQ ID NO: 1) and SEQ ID NO: 6 (corresponding to the amino acids 1021 to 1040 of SEQ ID NO: 1) were used as primers for PCR. Transcription was analyzed by RT-PCR, using RNA PCR Kit (AMV) Ver. 2.1 (Takara). The reaction was carried out in accordance with the attached manual using random nona-nucleotides as the primers for the reverse transcription reaction and the abovementioned primers as the primers for PCR. The samples were treated in the same manner without transcriptase in order to confirm that the RNA samples were not contaminated with DNA. After the reaction, electrophoresis was carried out on a 2% agarose gel, after which the gel was incubated in an ethidium bromide solution, then the resulting gel was photographed under UV light. Results are shown in Figure 1. This analysis indicated that the novel insecticidal protein gene of strain SSK-10 was evidently transcribed in tandem with CI production.

Example 6: Expression of novel insecticidal protein in E. coli and insecticidal activity of the protein

(1) Construction of vector for protein expression

The three bases (AGT) located immediately upstream of the translation initiation codon (ATG, 211 of SEQ ID NO: 1) of the novel gene were substituted with CAT to change this site into the recognition sequence (CATATG) for NdeI. A fragment extending from this NdeI site to the BamHI site (GGATCC, 3670 of SEQ ID NO: 1) located 7 bases downstream of the termination codon of the novel gene was inserted between the NdeI site and the BamHI site of pET-11a, a vector for protein expression (Stratagene). The vector to produce the novel insecticidal protein in E. coli was thus constructed.

(2) Expression of novel insecticidal protein in E. coli and purification of the protein

The vector constructed in (1) above was introduced into E. coli strain BL21 (DE3) for protein expression. Production of the novel insecticidal protein was carried out by inducing expression of the protein with isopropyl-$\beta$-D-thiogalactopyranoside (IPTG) in accordance with the attached manual. The E. coli bodies cultured in the presence of IPTG were confirmed to have produced the novel insecticidal protein of about 130 kd by SDS-polyacrylamide gel electrophoresis. This protein was produced in E. coli in the form of inclusions. The E. coli bodies were treated with lysozyme and DNase I, then centrifuged, and the inclusions were purified as a precipitate.

(3) Insecticidal activity of the novel insecticidal protein

a) Insecticidal activity of the novel insecticidal protein against Spodoptera litura

The novel insecticidal protein purified in (2) above was used as a test sample, and the spores prepared in Test Example 6 were added to the final spore concentration to 100 mg/L. Each sample was diluted to the specified concentration with a solution supplemented with a wetting agent, and cabbage leaf sections were sprayed with the solution. The sections were dried in plastic cups in air, and then third-instar larvae of Spodoptera litura were freed (5 larvae per cup). The cups were then covered and placed in an incubator at 25 °C, and life or death was observed after 3 days.
Results are shown in Table 9.

Table 9

| Insecticidal activity of the novel insecticidal protein against Spodoptera litura | | |
|---|---|---|
| Sample | Concentration of insecticidal protein (ppm) | Insect death rate (%) |
| Novel insecticidal protein | 100 | 0 |
| Novel insecticidal protein + spores* | 100 | 50 |
| Spores* | 0 | 0 |

It is known that because of the strong protease activity of the digestive juices of noctuids, insecticidal activity of the solubilized CIs against Spodoptera sp. is generally lower than that of the CIs before solubilization. Also, Bt insecticidal proteins which are produced by E. coli and do not have a crystalline structure are known to have lower insecticidal activity against noctuids than the CIs derived from the same gene produced by Bt bacteria (M. Keller et al., Insect Biochem. Molec. Biol., 26, 365, 1996). This phenomenon was also observed with CIs of strain SSK-10 as shown in Table 7, but the decrease in activity due to the solubilization was restored by the addition of the spores. The novel insecticidal protein produced in E. coli bodies alone had no insecticidal activity as shown in Table 9; however, the insecticidal activity was restored by the addition of the spores of strain SSK-10. Thus, it was concluded that the novel insecticidal protein regenerated the activity of the CIs of strain SSK-10.

b) Effect of the novel insecticidal protein on Bt-agent resistant Plutella xylostella

Effect of the novel insecticidal protein against Plutella xylostella (the resistant line) caught in Osaka Prefecture was compared with that against insects of the corresponding sensitive line. The dried product prepared by the method of Example 3 was used as the strain SSK-10 test sample. Each sample was diluted with a solution supplemented with a wetting agent to the specified concentration. Cabbage leaf sections were sprayed with the samples and dried in plastic cups in air, and then third-instar larvae of the resistant line or the sensitive line of Plutella xylostella were freed (5 larvae per cup). The cups were then covered and placed in an incubator at 25 °C, and life or death was observed after 3 days. Toaro-CT wettable powder (Towagosei Chem. KK) was used as the control chemical. Median lethal concentrations ($LC_{30}$) were calculated by the Probit method, and resistance ratios were calculated by dividing the $LC_{50}$ value for resistant line insects by the $LC_{50}$ value for sensitive line insects.
Results are shown in Table 10.

Table 10

| Insecticidal activity of the novel insecticidal protein against Bt-agent resistan Plutella Xylostella | |
| --- | --- |
| Sample | Resistance ratio |
| Novel Insecticidal protein | 0.35 |
| SSK-10 | 3.21 |
| Toaro CT wettable powder* (control) | 16.3 |

*Towagosei Chem. KK

This showed that the novel insecticidal protein had high insecticidal activity against Bt-agent resistant Plutella xylostella.

Examples of compositions of various formulations of the present invention are given as follows:

Formulation Example 1: Wettable powder

| CI- and spore-containing material of the present invention | 50% |
| --- | --- |
| Sorpol 807010% (anionic surfactant, trade name, Toho Chemical Industry Co., Ltd.) | 10% |
| Newkalgen WG-2 (anionic surfactant, trade name, Takemoto Oil & Fat Co., Ltd.) | 5% |
| Cellogen (carboxymethylcellulose, trade name, Daiichi-Kogyo Seiyaku Co., Ltd.) | 3% |
| Neutral anhydrous sodium sulfate | 16% |
| Radiolite #200 | 16% |

The components above are crushed and mixed homogeneously into a wettable powder.

For application, the wetting powder is diluted 500 to 4000 times and sprayed.

Formulation Example 2: Powder

| CI- and spore-containing material of the present invention | 3% |
| --- | --- |
| Carplex #80 (while carbon black, trade name, Shionogi & Co., Ltd.) | 1% |
| Diisopropyl phosphate | 2% |
| Talc | 94% |

The components above are crushed and mixed homogeneously into a powder.

Formulation Example 3: Flowable agent

| CI- and spore-containing material of the present invention | 30% |
|---|---|
| Polyethylene glycol | 5% |
| Xanthan gum | 0.2% |
| Newkalgen FS-1 (nonionic surfactant, trade name, Takemoto Oil & Fat Co., Ltd.) | 3% |
| Newkalgen FS-4 (anionic surfactant, trade name, Takemoto Oil & Fat Co., Ltd.) | 5% |
| Silicone | 0.1% |
| Water | 6.7% |

The components above, other than CI- and spore-containing material of the present invention, are dissolved homogeneously, the CI- and spore-containing material of the present invention is added, and then the resultant admixture is stirred thoroughly and crushed in moisture to obtain a flowable agent. For application, the flowable agent is diluted 25 to 2000 times and sprayed.

## SEQUENCE LISTING

SEQ ID NO: 1

SEQUENCE LENGTH: 3867

SEQUENCE TYPE: nucleic acid

STRANDNESS: double

TOPOLOGY: linear

MOLECULE TYPE: genomic DNA

HYPOTHETICAL: NO

ANTI-SENSE: NO

ORIGINAL SOURCE

    Organism: <u>Bacillus thuringiensis</u>

    Strain name: SSK-10

FEATURE

    Name/key: CDS

    Location: 211..3663

    Characteristics: P

    Name/key: mat peptide

    Location: 211..3660

    Characteristics: P

SEQUENCE DESCRIPTION

```
GAACCGAAGG TTTGTAAAAA AGAAATTGGA ATAAATACCA TCCATTTTTT CAAGAAAGAT    60
TTTTTTATTA GAAAGGAATC TTTCTTACAC AGGAGAATAC TAAGATTGAG AGTAGAGATA   120
TATAGATGTC AATACAATAA AGAGTCTGTC AGGTTTTTTG AAAGATATGA TTTGAACATG   180
TAATAGATTT ATAGTATAGG AGGAAAAAGT ATG AAT CGA AAT AAT CCA AAT GAA    234
                                  Met Asn Arg Asn Asn Pro Asn Glu
                                   1               5

TAT GAA ATT ATT GAT GCC CCC TAT TGT GGG TGT CCG TCA GAT GAT GAT    282
Tyr Glu Ile Ile Asp Ala Pro Tyr Cys Gly Cys Pro Ser Asp Asp Asp
       10                  15                  20

GTG AGG TAT CCT TTG GCA AGT GAC CCA AAT GCA GCG TTC CAA AAT ATG    330
Val Arg Tyr Pro Leu Ala Ser Asp Pro Asn Ala Ala Phe Gln Asn Met
 25                  30                  35                  40

AAC TAT AAA GAG TAT TTA CAA ACG TAT GAT GGA GAC TAC ACA GGT TCT    378
Asn Tyr Lys Glu Tyr Leu Gln Thr Tyr Asp Gly Asp Tyr Thr Gly Ser
               45                  50                  55

CTT ATC AAT CCT AAC TTA TCT ATT AAT CCT AGA GAT GTA CTA CAA ACA    426
```

```
Leu Ile Asn Pro Asn Leu Ser Ile Asn Pro Arg Asp Val Leu Gln Thr
              60                  65                  70
GGT ATT AAT ATT GTG GGA AGA ATA CTA GGG TTT TTA GGT GTT CCA TTT    474
Gly Ile Asn Ile Val Gly Arg Ile Leu Gly Phe Leu Gly Val Pro Phe
              75                  80                  85
GCG GGT CAA CTA GTT ACT TTC TAT ACC TTT CTC TTA AAT CAG TTG TGG    522
Ala Gly Gln Leu Val Thr Phe Tyr Thr Phe Leu Leu Asn Gln Leu Trp
           90                  95                  100
CCA ACT AAT GAT AAT GCA GTA TGG GAA GCT TTT ATG GCG CAA ATA GAA    570
Pro Thr Asn Asp Asn Ala Val Trp Glu Ala Phe Met Ala Gln Ile Glu
105                 110                 115                 120
GAG CTA ATC GAT CAA AAA ATA TCG GCG CAA GTA GTA AGG AAT GCA CTC    618
Glu Leu Ile Asp Gln Lys Ile Ser Ala Gln Val Val Arg Asn Ala Leu
                 125                 130                 135
GAT GAC TTA ACT GGA TTA CAC GAT TAT TAT GAG GAG TAT TTA GCA GCA    666
Asp Asp Leu Thr Gly Leu His Asp Tyr Tyr Glu Glu Tyr Leu Ala Ala
              140                 145                 150
TTA GAG GAG TGG CTG GAA AGA CCG AAC GGA GCA AGA GCT AAC TTA GTT    714
Leu Glu Glu Trp Leu Glu Arg Pro Asn Gly Ala Arg Ala Asn Leu Val
           155                 160                 165
ACA CAG AGG TTT GAA AAC CTG CAT ACT GCA TTT GTA ACT AGA ATG CCA    762
Thr Gln Arg Phe Glu Asn Leu His Thr Ala Phe Val Thr Arg Met Pro
           170                 175                 180
AGC TTT GGT ACG GGT CCT GGT AGT CAA AGA GAT GCG GTA GCG TTG TTG    810
Ser Phe Gly Thr Gly Pro Gly Ser Gln Arg Asp Ala Val Ala Leu Leu
185                 190                 195                 200
ACG GTA TAT GCA CAA GCA GCG AAT TTG CAT TTG TTA TTA TTA AAA GAT    858
Thr Val Tyr Ala Gln Ala Ala Asn Leu His Leu Leu Leu Leu Lys Asp
                 205                 210                 215
GCA GAA ATC TAT GGG GCA AGA TGG GGA CTT CAA CAA GGG CAA ATT AAC    906
Ala Glu Ile Tyr Gly Ala Arg Trp Gly Leu Gln Gln Gly Gln Ile Asn
                 220                 225                 230
TTA TAT TTT AAT GCT CAA CAA GAA CGT ACT CGA ATT TAT ACC AAT CAT    954
Leu Tyr Phe Asn Ala Gln Gln Glu Arg Thr Arg Ile Tyr Thr Asn His
              235                 240                 245
TGC GTG GAA ACA TAT AAT AGA GGA TTA GAA GAT GTA AGA GGA ACA AAT   1002
Cys Val Glu Thr Tyr Asn Arg Gly Leu Glu Asp Val Arg Gly Thr Asn
```

17

```
        250                      255                      260
ACA GAA AGT TGG TTA AAT TAC CAT CGA TTC CGT AGA GAG ATG ACA TTA   1050
Thr Glu Ser Trp Leu Asn Tyr His Arg Phe Arg Arg Glu Met Thr Leu
265                      270                      275            280
ATG GCA ATG GAT TTA GTG GCC CTA TTC CCA TTC TAT AAT GTG CGA CAA   1098
Met Ala Met Asp Leu Val Ala Leu Phe Pro Phe Tyr Asn Val Arg Gln
                    285                      290                      295
TAT CCA AAT GGG GCA AAT CCA CAG CTT ACA CGT GAA ATA TAT ACA GAT   1146
Tyr Pro Asn Gly Ala Asn Pro Gln Leu Thr Arg Glu Ile Tyr Thr Asp
                    300                      305                      310
CCA ATC GTA TAT AAT CCA CCA GCT AAT CAG GGA ATT TGC CGA CGT TGG   1194
Pro Ile Val Tyr Asn Pro Pro Ala Asn Gln Gly Ile Cys Arg Arg Trp
                    315                      320                      325
GGG AAT AAT CCG TAT AAT ACA TTT TCT GAA CTT GAA AAT GCT TTT ATT   1242
Gly Asn Asn Pro Tyr Asn Thr Phe Ser Glu Leu Glu Asn Ala Phe Ile
                    330                      335                      340
CGC CCG CCA CAT CTT TTT GAA AGG TTG AAC AGA TTA ACT ATT TCT AGA   1290
Arg Pro Pro His Leu Phe Glu Arg Leu Asn Arg Leu Thr Ile Ser Arg
345                      350                      355                      360
AAC CGA TAT ACA GCT CCA ACA ACT AAT AGC TTC CTA GAC TAT TGG TCA   1338
Asn Arg Tyr Thr Ala Pro Thr Thr Asn Ser Phe Leu Asp Tyr Trp Ser
                    365                      370                      375
GGT CAT ACT TTA CAA AGC CAA CAT GCA AAT AAC CCG ACG ACA TAT GAA   1386
Gly His Thr Leu Gln Ser Gln His Ala Asn Asn Pro Thr Thr Tyr Glu
                    380                      385                      390
ACT AGT TAC GGT CAG ATT ACC TCT AAC ACA CGT TTA TTC AAT ACG ACT   1434
Thr Ser Tyr Gly Gln Ile Thr Ser Asn Thr Arg Leu Phe Asn Thr Thr
                    395                      400                      405
AAT GGA GCC CGT GCA ATA GAT TCA AGG GCA AGA AAT TTT GGT AAC TTA   1482
Asn Gly Ala Arg Ala Ile Asp Ser Arg Ala Arg Asn Phe Gly Asn Leu
                410                      415                      420
TAC GCT AAT TTG TAT GGC GTT AGC AGC TTG AAC ATT TTC CCA ACA GGT   1530
Tyr Ala Asn Leu Tyr Gly Val Ser Ser Leu Asn Ile Phe Pro Thr Gly
425                      430                      435                      440
GTG ATG AGT GAA ATC ACC AAT GCA GCT AAT ACG TGT CGG CAA GAC CTT   1578
Val Met Ser Glu Ile Thr Asn Ala Ala Asn Thr Cys Arg Gln Asp Leu
                    445                      450                      455
```

```
ACT ACA ACT GAA GAA CTA CCA CTA GAG AAT AAT AAT TTT AAT CTT TTA   1626
Thr Thr Thr Glu Glu Leu Pro Leu Glu Asn Asn Asn Phe Asn Leu Leu
                460                 465                 470
TCT CAT GTT ACT TTC TTA CGC TTC AAT ACT ACT CAG GGT GGC CCC CTT   1674
Ser His Val Thr Phe Leu Arg Phe Asn Thr Thr Gln Gly Gly Pro Leu
                475                 480                 485
GCA ACT CTA GGG TTT GTA CCC ACA TAT GTG TGG ACA CGT GAA GAT GTA   1722
Ala Thr Leu Gly Phe Val Pro Thr Tyr Val Trp Thr Arg Glu Asp Val
        490                 495                 500
GAT TTT ACG AAC ACA ATT ACT GCG GAT AGA ATT ACA CAA CTA CCA TGG   1770
Asp Phe Thr Asn Thr Ile Thr Ala Asp Arg Ile Thr Gln Leu Pro Trp
505                 510                 515                 520
GTA AAG GCA TCT GAA ATA GGT GGG GGT ACT ACT GTC GTG AAA GGT CCA   1818
Val Lys Ala Ser Glu Ile Gly Gly Gly Thr Thr Val Val Lys Gly Pro
                525                 530                 535
GGA TTT ACA GGA GGG GAT ATA CTT CGA AGA ACG GAC GGT GGT GCA GTT   1866
Gly Phe Thr Gly Gly Asp Ile Leu Arg Arg Thr Asp Gly Gly Ala Val
                540                 545                 550
GGA ACG ATT AGA GCT AAT GTT AAT GCC CCA TTA ACA CAA CAA TAT CGT   1914
Gly Thr Ile Arg Ala Asn Val Asn Ala Pro Leu Thr Gln Gln Tyr Arg
                555                 560                 565
ATA AGA TTA CGC TAT GCT TCG ACA ACA AGT TTT GTT GTT AAT TTA TTT   1962
Ile Arg Leu Arg Tyr Ala Ser Thr Thr Ser Phe Val Val Asn Leu Phe
        570                 575                 580
GTT AAT AAT AGT GCG GCT GGC TTT ACT TTA CCG AGT ACA ATG GCT CAA   2010
Val Asn Asn Ser Ala Ala Gly Phe Thr Leu Pro Ser Thr Met Ala Gln
585                 590                 595                 600
AAT GGT TCT TTA ACA TAC GAG TCG TTT AAT ACC TTA GAG GTA ACT CAT   2058
Asn Gly Ser Leu Thr Tyr Glu Ser Phe Asn Thr Leu Glu Val Thr His
                605                 610                 615
ACT ATT AGA TTT TCA CAG TCA GAT ACT ACA CTT AGG TTG AAT ATA TTC   2106
Thr Ile Arg Phe Ser Gln Ser Asp Thr Thr Leu Arg Leu Asn Ile Phe
                620                 625                 630
CCG TCT ATC TCT GGT CAA GAA GTG TAT GTA GAT AAA CTT GAA ATC GTT   2154
Pro Ser Ile Ser Gly Gln Glu Val Tyr Val Asp Lys Leu Glu Ile Val
                635                 640                 645
CCA ATT AAC CCG ACA CGA GAA GCG GAA GAA GAT TTA GAA GAT GCA AAG   2202
```

```
Pro Ile Asn Pro Thr Arg Glu Ala Glu Glu Asp Leu Glu Asp Ala Lys
    650                 655                 660
AAA GCG GTG GCG AGC TTG TTT ACA CGT ACA AGG GAT GGA TTA CAG GTA   2250
Lys Ala Val Ala Ser Leu Phe Thr Arg Thr Arg Asp Gly Leu Gln Val
665                 670                 675                 680
AAT GTG ACA GAT TAC CAA GTC GAT CAG GCG GCA AAT TTA GTG TCG TGC   2298
Asn Val Thr Asp Tyr Gln Val Asp Gln Ala Ala Asn Leu Val Ser Cys
                685                 690                 695
TTA TCA GAT GAA CAA TAT GGG CAT GAT AAA AAG ATG TTA TTG GAA GCC   2346
Leu Ser Asp Glu Gln Tyr Gly His Asp Lys Lys Met Leu Leu Glu Ala
                700                 705                 710
GTA CGC GCA GCA AAA CGC CTC AGC CGC GAA CGC AAC TTA CTT CAA GAT   2394
Val Arg Ala Ala Lys Arg Leu Ser Arg Glu Arg Asn Leu Leu Gln Asp
                715                 720                 725
CCA GAT TTT AAT GAA ATA AAT AGC ACA GAA GAA AAT GGC TGG AAG GCA   2442
Pro Asp Phe Asn Glu Ile Asn Ser Thr Glu Glu Asn Gly Trp Lys Ala
                730                 735                 740
AGT AAC GGT GTT ACT ATT AGC GAG GGC GGT CCA TTC TTT AAA GGT CGT   2490
Ser Asn Gly Val Thr Ile Ser Glu Gly Gly Pro Phe Phe Lys Gly Arg
745                 750                 755                 760
GCA CTT CAG TTA GCA AGC GCA CGT GAA AAT TAC CCA ACA TAC ATC TAT   2538
Ala Leu Gln Leu Ala Ser Ala Arg Glu Asn Tyr Pro Thr Tyr Ile Tyr
                765                 770                 775
CAA AAG GTA GAT GCA TCG ACG TTA AAA CCT TAT ACA CGA TAT AAA CTA   2586
Gln Lys Val Asp Ala Ser Thr Leu Lys Pro Tyr Thr Arg Tyr Lys Leu
                780                 785                 790
GAT GGA TTT GTG CAA AGT AGT CAA GAT TTA GAA ATT GAC CTC ATT CAT   2634
Asp Gly Phe Val Gln Ser Ser Gln Asp Leu Glu Ile Asp Leu Ile His
                795                 800                 805
CAT CAT AAA GTC CAC CTC GTG AAA AAT GTA CCA GAT AAT TTA GTA TCT   2682
His His Lys Val His Leu Val Lys Asn Val Pro Asp Asn Leu Val Ser
                810                 815                 820
GAT ACT TAT TCT GAT GGC TCA TGT AGT GGA ATT AAC CGT TGT GAG GAA   2730
Asp Thr Tyr Ser Asp Gly Ser Cys Ser Gly Ile Asn Arg Cys Glu Glu
825                 830                 835                 840
CAA CAT CAG GTA GAT GTG CAG CTA GAT GCG GAG GAT CAT CCA AAG GAT   2778
Gln His Gln Val Asp Val Gln Leu Asp Ala Glu Asp His Pro Lys Asp
```

```
                    845                        850                        855
TGT TGT GAA GCG GCT CAA ACA CAT GAG TTT TCT TCC TAT ATT CAT ACA   2826
Cys Cys Glu Ala Ala Gln Thr His Glu Phe Ser Ser Tyr Ile His Thr
                    860                        865                        870
GGT GAT CTA AAT GCA AGT GTA GAT CAA GGC ATT TGG GTT GTA TTG CAG   2874
Gly Asp Leu Asn Ala Ser Val Asp Gln Gly Ile Trp Val Val Leu Gln
          875                        880                        885
GTT CGA ACA ACA GAT GGT TAT GCG ACG TTA GGA AAT CTT GAA TTG GTA   2922
Val Arg Thr Thr Asp Gly Tyr Ala Thr Leu Gly Asn Leu Glu Leu Val
          890                        895                        900
GAG GTT GGT CCA TTA TCG GGT GAA TCT TTA GAA CGA GAA CAA AGA GAT   2970
Glu Val Gly Pro Leu Ser Gly Glu Ser Leu Glu Arg Glu Gln Arg Asp
905                        910                        915                        920
AAT GCG AAA TGG AAT GAA GAG GTA GGA AGA AAG CGT GCA GAA ACA GAT   3018
Asn Ala Lys Trp Asn Glu Glu Val Gly Arg Lys Arg Ala Glu Thr Asp
                    925                        930                        935
CGC ATA TAT CAA GAT GCG AAA CAA GCA ATT AAC CAT CTA TTT GTA GAC   3066
Arg Ile Tyr Gln Asp Ala Lys Gln Ala Ile Asn His Leu Phe Val Asp
                    940                        945                        950
TAT CAA GAT CAA CAA TTA AGT CCA GAG GTA GGG ATG GCG GAT ATT ATT   3114
Tyr Gln Asp Gln Gln Leu Ser Pro Glu Val Gly Met Ala Asp Ile Ile
          955                        960                        965
GAT GCT CAA AAT CTT ATC GCA TCA ATT TCA GAT GTA TAT AGC GAT GCA   3162
Asp Ala Gln Asn Leu Ile Ala Ser Ile Ser Asp Val Tyr Ser Asp Ala
          970                        975                        980
GTA CTG CAA ATC CCT GGG ATT AAC TAC GAG ATG TAT ACA GAG TTA TCC   3210
Val Leu Gln Ile Pro Gly Ile Asn Tyr Glu Met Tyr Thr Glu Leu Ser
985                        990                        995                        1000
AAT CGA TTA CAA CAA GCA TCG TAT CTG TAT ACG TCT CGA AAT GTC GTG   3258
Asn Arg Leu Gln Gln Ala Ser Tyr Leu Tyr Thr Ser Arg Asn Val Val
                    1005                       1010                       1015
CAA AAT GGG GAC TTT AAC AGT GGT TTA GAT AGT TGG AAT GCA ACA ACT   3306
Gln Asn Gly Asp Phe Asn Ser Gly Leu Asp Ser Trp Asn Ala Thr Thr
                    1020                       1025                       1030
GAT ACA GCT GTT CAG CAG GAT GGC AAT ATG CAT TTC TTA GTT CTT TCC   3354
Asp Thr Ala Val Gln Gln Asp Gly Asn Met His Phe Leu Val Leu Ser
          1035                       1040                       1045
```

```
CAT TGG GAT GCA CAA GTT TCT CAA CAA TTT AGA GTA CAG CCG AAT TGT   3402
His Trp Asp Ala Gln Val Ser Gln Gln Phe Arg Val Gln Pro Asn Cys
    1050                1055                1060
AAA TAT GTG TTA CGT GTG ACA GCG AAG AAA GTA GGG AAC GGA GAT GGA   3450
Lys Tyr Val Leu Arg Val Thr Ala Lys Lys Val Gly Asn Gly Asp Gly
1065                1070                1075                1080
TAT GTT ACG ATC CAA GAT GGC GCT CAT CAC CGA GAA ACA CTG ACA TTC   3498
Tyr Val Thr Ile Gln Asp Gly Ala His His Arg Glu Thr Leu Thr Phe
                1085                1090                1095
AAT GCA TGT GAC TAC GAT GTA AAT GGT ACG CAT GTA AAT GAT AAT TCG   3546
Asn Ala Cys Asp Tyr Asp Val Asn Gly Thr His Val Asn Asp Asn Ser
            1100                1105                1110
TAT ATT ACA AAA GAA TTG GTG TTC TAT CCA AAG ACG GAA CAT ATG TGG   3594
Tyr Ile Thr Lys Glu Leu Val Phe Tyr Pro Lys Thr Glu His Met Trp
        1115                1120                1125
GTA GAG GTA AGT GAA ACA GAA GGT ACC TTC TAT ATA GAC AGC ATT GAG   3642
Val Glu Val Ser Glu Thr Glu Gly Thr Phe Tyr Ile Asp Ser Ile Glu
        1130                1135                1140
TTC ATT GAA ACA CAA GAG TAGAAGAGGG GATCCTAACG TATAGCAACT          3690
Phe Ile Glu Thr Gln Glu
1145                1150
ATGAGGGGAC ACTCTGTATA AATAAAGATT AAAAAGAATA GAAAATGAAT AGAACCCCCT 3750
ACTGGTCTTT TTACCAGTAG GGGGTTTTTC ACATAAAAAA ATGGATTTGT TTACTAAGGT 3810
GTATAAAAAA CGGAATACCT GATAGAAAAA AGGGAGTACC TTATAAAGAA AGAATTC     3867
```

SEQ ID NO: 2
SEQUENCE LENGTH: 1150
SEQUENCE TYPE: amino acid
TOPOLOGY: linear
MOLECULE TYPE: Protein
ORIGINAL SOURCE
    Organism: <u>Bacillus thuringiensis</u>
    Strain name: SSK-10
SEQUENCE DESCRIPTION

```
Met Asn Arg Asn Asn Pro Asn Glu Tyr Glu Ile Ile Asp Ala Pro Tyr
 1               5                   10                  15

Cys Gly Cys Pro Ser Asp Asp Asp Val Arg Tyr Pro Leu Ala Ser Asp
             20                  25                  30

Pro Asn Ala Ala Phe Gln Asn Met Asn Tyr Lys Glu Tyr Leu Gln Thr
             35                  40                  45

Tyr Asp Gly Asp Tyr Thr Gly Ser Leu Ile Asn Pro Asn Leu Ser Ile
     50                  55                  60

Asn Pro Arg Asp Val Leu Gln Thr Gly Ile Asn Ile Val Gly Arg Ile
 65                  70                  75                  80

Leu Gly Phe Leu Gly Val Pro Phe Ala Gly Gln Leu Val Thr Phe Tyr
                 85                  90                  95

Thr Phe Leu Leu Asn Gln Leu Trp Pro Thr Asn Asp Asn Ala Val Trp
                 100                 105                 110

Glu Ala Phe Met Ala Gln Ile Glu Glu Leu Ile Asp Gln Lys Ile Ser
             115                 120                 125

Ala Gln Val Val Arg Asn Ala Leu Asp Asp Leu Thr Gly Leu His Asp
         130                 135                 140

Tyr Tyr Glu Glu Tyr Leu Ala Ala Leu Glu Glu Trp Leu Glu Arg Pro
145                 150                 155                 160

Asn Gly Ala Arg Ala Asn Leu Val Thr Gln Arg Phe Glu Asn Leu His
             165                 170                 175

Thr Ala Phe Val Thr Arg Met Pro Ser Phe Gly Thr Gly Pro Gly Ser
             180                 185                 190

Gln Arg Asp Ala Val Ala Leu Leu Thr Val Tyr Ala Gln Ala Ala Asn
         195                 200                 205

Leu His Leu Leu Leu Leu Lys Asp Ala Glu Ile Tyr Gly Ala Arg Trp
     210                 215                 220
```

```
Gly Leu Gln Gln Gly Gln Ile Asn Leu Tyr Phe Asn Ala Gln Gln Glu
225                 230             235                 240
Arg Thr Arg Ile Tyr Thr Asn His Cys Val Glu Thr Tyr Asn Arg Gly
                245             250             255
Leu Glu Asp Val Arg Gly Thr Asn Thr Glu Ser Trp Leu Asn Tyr His
            260             265             270
Arg Phe Arg Arg Glu Met Thr Leu Met Ala Met Asp Leu Val Ala Leu
            275             280             285
Phe Pro Phe Tyr Asn Val Arg Gln Tyr Pro Asn Gly Ala Asn Pro Gln
            290             295             300
Leu Thr Arg Glu Ile Tyr Thr Asp Pro Ile Val Tyr Asn Pro Pro Ala
305             310             315                 320
Asn Gln Gly Ile Cys Arg Arg Trp Gly Asn Asn Pro Tyr Asn Thr Phe
                325             330             335
Ser Glu Leu Glu Asn Ala Phe Ile Arg Pro Pro His Leu Phe Glu Arg
            340             345             350
Leu Asn Arg Leu Thr Ile Ser Arg Asn Arg Tyr Thr Ala Pro Thr Thr
            355             360             365
Asn Ser Phe Leu Asp Tyr Trp Ser Gly His Thr Leu Gln Ser Gln His
            370             375             380
Ala Asn Asn Pro Thr Thr Tyr Glu Thr Ser Tyr Gly Gln Ile Thr Ser
385             390             395                 400
Asn Thr Arg Leu Phe Asn Thr Thr Asn Gly Ala Arg Ala Ile Asp Ser
            405             410             415
Arg Ala Arg Asn Phe Gly Asn Leu Tyr Ala Asn Leu Tyr Gly Val Ser
            420             425             430
Ser Leu Asn Ile Phe Pro Thr Gly Val Met Ser Glu Ile Thr Asn Ala
            435             440             445
Ala Asn Thr Cys Arg Gln Asp Leu Thr Thr Thr Glu Glu Leu Pro Leu
            450             455             460
Glu Asn Asn Asn Phe Asn Leu Leu Ser His Val Thr Phe Leu Arg Phe
465                 470             475                 480
Asn Thr Thr Gln Gly Gly Pro Leu Ala Thr Leu Gly Phe Val Pro Thr
                485             490             495
Tyr Val Trp Thr Arg Glu Asp Val Asp Phe Thr Asn Thr Ile Thr Ala
                500             505             510
Asp Arg Ile Thr Gln Leu Pro Trp Val Lys Ala Ser Glu Ile Gly Gly
```

```
                515                    520                    525
        Gly Thr Thr Val Val Lys Gly Pro Gly Phe Thr Gly Gly Asp Ile Leu
            530                    535                    540
        Arg Arg Thr Asp Gly Gly Ala Val Gly Thr Ile Arg Ala Asn Val Asn
        545                    550                    555                    560
        Ala Pro Leu Thr Gln Gln Tyr Arg Ile Arg Leu Arg Tyr Ala Ser Thr
                            565                    570                    575
        Thr Ser Phe Val Val Asn Leu Phe Val Asn Asn Ser Ala Ala Gly Phe
                    580                    585                    590
        Thr Leu Pro Ser Thr Met Ala Gln Asn Gly Ser Leu Thr Tyr Glu Ser
                    595                    600                    605
        Phe Asn Thr Leu Glu Val Thr His Thr Ile Arg Phe Ser Gln Ser Asp
            610                    615                    620
        Thr Thr Leu Arg Leu Asn Ile Phe Pro Ser Ile Ser Gly Gln Glu Val
        625                    630                    635                    640
        Tyr Val Asp Lys Leu Glu Ile Val Pro Ile Asn Pro Thr Arg Glu Ala
                            645                    650                    655
        Glu Glu Asp Leu Glu Asp Ala Lys Lys Ala Val Ala Ser Leu Phe Thr
                    660                    665                    670
        Arg Thr Arg Asp Gly Leu Gln Val Asn Val Thr Asp Tyr Gln Val Asp
                    675                    680                    685
        Gln Ala Ala Asn Leu Val Ser Cys Leu Ser Asp Glu Gln Tyr Gly His
            690                    695                    700
        Asp Lys Lys Met Leu Leu Glu Ala Val Arg Ala Ala Lys Arg Leu Ser
        705                    710                    715                    720
        Arg Glu Arg Asn Leu Leu Gln Asp Pro Asp Phe Asn Glu Ile Asn Ser
                            725                    730                    735
        Thr Glu Glu Asn Gly Trp Lys Ala Ser Asn Gly Val Thr Ile Ser Glu
                    740                    745                    750
        Gly Gly Pro Phe Phe Lys Gly Arg Ala Leu Gln Leu Ala Ser Ala Arg
                    755                    760                    765
        Glu Asn Tyr Pro Thr Tyr Ile Tyr Gln Lys Val Asp Ala Ser Thr Leu
                    770                    775                    780
        Lys Pro Tyr Thr Arg Tyr Lys Leu Asp Gly Phe Val Gln Ser Ser Gln
        785                    790                    795                    800
        Asp Leu Glu Ile Asp Leu Ile His His His Lys Val His Leu Val Lys
                            805                    810                    815
```

```
Asn Val Pro Asp Asn Leu Val Ser Asp Thr Tyr Ser Asp Gly Ser Cys
                820                 825                 830
Ser Gly Ile Asn Arg Cys Glu Glu Gln His Gln Val Asp Val Gln Leu
            835                 840                 845
Asp Ala Glu Asp His Pro Lys Asp Cys Cys Glu Ala Ala Gln Thr His
        850                 855                 860
Glu Phe Ser Ser Tyr Ile His Thr Gly Asp Leu Asn Ala Ser Val Asp
865                 870                 875                 880
Gln Gly Ile Trp Val Val Leu Gln Val Arg Thr Thr Asp Gly Tyr Ala
                885                 890                 895
Thr Leu Gly Asn Leu Glu Leu Val Glu Val Gly Pro Leu Ser Gly Glu
            900                 905                 910
Ser Leu Glu Arg Glu Gln Arg Asp Asn Ala Lys Trp Asn Glu Glu Val
            915                 920                 925
Gly Arg Lys Arg Ala Glu Thr Asp Arg Ile Tyr Gln Asp Ala Lys Gln
        930                 935                 940
Ala Ile Asn His Leu Phe Val Asp Tyr Gln Asp Gln Gln Leu Ser Pro
945                 950                 955                 960
Glu Val Gly Met Ala Asp Ile Ile Asp Ala Gln Asn Leu Ile Ala Ser
                965                 970                 975
Ile Ser Asp Val Tyr Ser Asp Ala Val Leu Gln Ile Pro Gly Ile Asn
                980                 985                 990
Tyr Glu Met Tyr Thr Glu Leu Ser Asn Arg Leu Gln Gln Ala Ser Tyr
            995                 1000                1005
Leu Tyr Thr Ser Arg Asn Val Val Gln Asn Gly Asp Phe Asn Ser Gly
        1010                1015                1020
Leu Asp Ser Trp Asn Ala Thr Thr Asp Thr Ala Val Gln Gln Asp Gly
1025                1030                1035                1040
Asn Met His Phe Leu Val Leu Ser His Trp Asp Ala Gln Val Ser Gln
                1045                1050                1055
Gln Phe Arg Val Gln Pro Asn Cys Lys Tyr Val Leu Arg Val Thr Ala
            1060                1065                1070
Lys Lys Val Gly Asn Gly Asp Gly Tyr Val Thr Ile Gln Asp Gly Ala
        1075                1080                1085
His His Arg Glu Thr Leu Thr Phe Asn Ala Cys Asp Tyr Asp Val Asn
        1090                1095                1100
Gly Thr His Val Asn Asp Asn Ser Tyr Ile Thr Lys Glu Leu Val Phe
```

```
     1105                  1110                  1115                  1120
     Tyr Pro Lys Thr Glu His Met Trp Val Glu Val Ser Glu Thr Glu Gly
                          1125                  1130                  1135
     Thr Phe Tyr Ile Asp Ser Ile Glu Phe Ile Glu Thr Gln Glu
                   1140                  1145                  1150
```

SEQ ID NO: 3

SEQUENCE LENGTH: 21

SEQUENCE TYPE: nucleic acid

STRANDNESS: single

TOPOLOGY: linear

MOLECULE TYPE: Other nucleic acid/synthetic DNA

SEQUENCE DESCRIPTION

AGRWCCAGGA TTTAYAGGAG G

SEQ ID NO: 4

SEQUENCE LENGTH: 20

SEQUENCE TYPE:nucleic acid

STRANDNESS: single

TOPOLOGY: linear

MOLECULE TYPE: Other nucleic acid/synthetic DNA

SEQUENCE DESCRIPTION

CCCCTGTATC AATATAGRAA

SEQ ID NO: 5

SEQUENCE LENGTH: 20

SEQUENCE TYPE: nucleic acid

STRANDNESS: single

TOPOLOGY: linear

MOLECULE TYPE: Other nucleic acid/synthetic DNA

SEQUENCE DESCRIPTION

TAGGTGTTCC ATTTGCGGGT

SEQ ID NO: 6

SEQUENCE LENGTH: 20

SEQUENCE TYPE: nucleic acid

STRANDNESS: single

TOPOLOGY: linear

MOLECULE TYPE: Other nucleic acid/synthetic DNA

SEQUENCE DESCRIPTION

TCTCTACGGA ATCGATGGTA

**Claims**

1.  A strain SSK-10 which was deposited under the access number of FERM BP-6162.

2.  A protein comprising the following amino acid sequence(a) or (b):

    (a) an amino acid sequence of SEQ ID NO: 2, or
    (b) an amino acid sequence of SEQ ID NO: 2 which has insecticidal activity wherein one or more amino acids in SEQ ID NO: 2 are substituted or deleted, or one or more amino acids are added or inserted into the amino acid sequence of SEQ ID NO: 2.

3.  A nucleotide sequence encoding a protein according to claim 2.

4.  A nucleotide sequence according to claim 3 which has the nucleotide sequence of SEQ ID NO: 1.

5.  A vector comprising a nucleotide sequence according to claim 3.

6.  A host cell comprising a nucleotide sequence according to claim 3 or a vector according to claim 5.

7.  A host cell according to claim 6 which is a microorganism.

8.  A host cell according to claim 6 which is Escherichia coli.

9.  A protein according to claim 2 which is in the form of crystalline protein inclusions.

10. A protein according to claim 2 which is in the form of solubilized crystalline protein inclusions.

11. A protein according to claim 2 which is produced by a strain according to claim 1 or a host cell accoring to claim 6.

12. An agent for exterminating insect pests comprising a strain according to claim 1, a host cell according to claim 6 and/or a protein according to claim 2 as an active ingredient.

13. An agent for exterminating insect pests comprising a protein according to claim 2 and spores of the strain according to claim 1.

14. An agent for exterminating insect pests comprising crystalline protein inclusions according to claim 9 and spores of a strain according to claim 1.

15. An agent for exterminating insect pests comprising solubilized crystalline protein inclusions according to claim 10 and spores of a strain according to claim 1.

16. A method for exterminating insect pests comprising treating plants which have been or could be damaged by the insect pests with a strain according to claim 1, a microorganism according to claim 7 and/or a protein according to claim 2.

17. A method according to claim 16 wherein said insect pests are classified into the Lepidoptera, Coleoptera, or Diptera.

18. A method according to claim 16 wherein the insects are those selected from the group consisting of Spodoptera litura, Plutella xylostella, and Culex pipiens.

19. A plant cell comprising a nucleotide sequence according to claim 3 or a vector according to claim 5.

20. A plant cell according to claim 19 which is regenerated into a plant which is capable of producing seeds.

21. A seed comprising a nucleotide sequence according to claim 3 or a vector according to claim 5.

22. A seed according to claim 21 which can be grown into a plant which is capable of producing seeds.

23. A plant comprising a nucleotide sequence according to claim 3 or a vector according to claim 5.

24. A plant according to claim 23 which is capable of producing seeds.

25. A plant which can be obtained by germinating a seed of claim 21.

F I G. 1

## INTERNATIONAL SEARCH REPORT

| | |
|---|---|
| International application No. | |
| PCT/JP97/04530 | |

**A. CLASSIFICATION OF SUBJECT MATTER**
Int.Cl⁶ C12N15/32, C12N1/21, C07K14/325, C12N5/00, A01N63/00,
A01H1/00, A01H5/00
According to International Patent Classification (IPC) or to both national classification and IPC

**B. FIELDS SEARCHED**

Minimum documentation searched (classification system followed by classification symbols)
Int.Cl⁶ C12N15/32, C12N1/21, C07K14/325, C12N5/00, A01N63/00,
A01H1/00, A01H5/00

Documentation searched other than minimum documentation to the extent that such documents are included in the fields searched

Electronic data base consulted during the international search (name of data base and, where practicable, search terms used)
BIOSIS (DIALOG), WPI (DIALOG), DDBJ (GENETYX)

**C. DOCUMENTS CONSIDERED TO BE RELEVANT**

| Category* | Citation of document, with indication, where appropriate, of the relevant passages | Relevant to claim No. |
|---|---|---|
| X<br>Y | Bart, L. et al. "A Bacillus thuringiensis Insecticidal Crystal Protein with a High Activity against Members of the Family Noctuidae" Appl. Environ. Microbiol. (1996, Jan) Vol. 62, No. 1, p.80-86 | 2-3, 5-11<br>12-25 |
| Y | William, J. M. et al. "Development of Bacillus thuringiensis Cry IC Resistance by Spodoptera exigua" Apl. Environ. Microbiol. (1995) Vol. 61, No. 6, p.2086-2092 | 12-18 |
| Y | JP, 63-17687, A (Toagosei Chemical Industry Co., Ltd.), January 25, 1988 (25. 01. 88) (Family: none) | 12-18 |
| Y | JP, 5-507194, A (Plant Genetic Systems, N.V.), October 21, 1993 (21. 10. 93) & WO, 9116433, A & AU, 9177526, A & EP, 528857, A1 & US, 5466597, A | 19-25 |

☐ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | | |
| | | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| March 3, 1998 (03. 03. 98) | March 10, 1998 (10. 03. 98) |

| Name and mailing address of the ISA/ | Authorized officer |
|---|---|
| Japanese Patent Office | |
| Facsimile No. | Telephone No. |

Form PCT/ISA/210 (second sheet) (July 1992)